(19) Europäisches Patentamt / European Patent Office / Office européen des brevets

(11) **EP 2 068 973 B1**

(12) **EUROPÄISCHE PATENTSCHRIFT**

(45) Veröffentlichungstag und Bekanntmachung des Hinweises auf die Patenterteilung:
**10.02.2016 Patentblatt 2016/06**

(21) Anmeldenummer: **07818381.1**

(22) Anmeldetag: **25.09.2007**

(51) Int Cl.:
**A61M 1/36** (2006.01)

(86) Internationale Anmeldenummer:
**PCT/EP2007/008296**

(87) Internationale Veröffentlichungsnummer:
**WO 2008/037409 (03.04.2008 Gazette 2008/14)**

(54) **VERFAHREN UND VORRICHTUNG ZUR ERKENNUNG VON LUFT IN EINEM FLÜSSIGKEITSSYSTEM, INSBESONDERE IN EINEM EXTRAKORPORALEN BLUTKREISLAUF EINER BLUTBEHANDLUNGSVORRICHTUNG**

METHOD AND DEVICE FOR DETECTING AIR IN A FLUID SYSTEM, IN PARTICULAR IN AN EXTRACORPOREAL BLOOD CIRCUIT OF A BLOOD TREATMENT DEVICE

PROCÉDÉ ET DISPOSITIF DE DÉTECTION D'AIR DANS UN CIRCUIT DE FLUIDES, EN PARTICULIER DANS UN CIRCUIT SANGUIN EXTRACORPOREL D'UN DISPOSITIF DE TRAITEMENT SANGUIN

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **26.09.2006 DE 102006045452**

(43) Veröffentlichungstag der Anmeldung:
**17.06.2009 Patentblatt 2009/25**

(73) Patentinhaber: **Fresenius Medical Care Deutschland GmbH**
**61352 Bad Homburg v.d.H. (DE)**

(72) Erfinder: **KOPPERSCHMIDT, Pascal**
**97456 Dittelbrunn (DE)**

(74) Vertreter: **Oppermann, Frank et al**
**OANDO Oppermann & Oppermann LLP**
**Washingtonstrasse 75**
**65189 Wiesbaden (DE)**

(56) Entgegenhaltungen:
**WO-A-98/00685     US-A1- 2002 174 721**

**Beschreibung**

Die Erfindung bezieht sich auf ein Verfahren und eine Vorrichtung zur Erkennung des Eintritts von Luft in ein Flüssigkeitssystem, insbesondere in einen extrakorporalen Blutkreislauf einer Blutbehandlungsvorrichtung, der einen zu einer Blutbehandlungseinheit führenden arteriellen Zweig und einen von der Blutbehandlungseinheit abgehenden venösen Zweig aufweist. Darüber hinaus betrifft die Erfindung eine extrakorporale Blutbehandlungsvorrichtung, insbesondere Dialysevorrichtung, mit einer Vorrichtung zur Erkennung eines Lufteintritts in den extrakorporalen Blutkreislauf.

Auf dem Gebiet der Medizintechnik sind eine Vielzahl von Einrichtungen bekannt, mit denen über eine Schlauchleitung Patienten Flüssigkeiten entnommen oder Flüssigkeiten den Patienten zugeführt werden können. Dabei erfolgt der Zugang zu den Patienten im Allgemeinen mit Kathetern zum Einführen in Körperorgane oder Kanülen zum Punktieren von Gefäßen.

Bei Verfahren der chronischen Blutreinigungstherapie wie Hämodialyse, Hämofiltration und Hämodiafiltration wird Blut eines Patienten über einen extrakorporalen Blutkreislauf geleitet, der einen zu dem als Dialysator ausgebildeten Blutreinigungselement führenden arteriellen Zweig und einen von dem Dialysator abgehenden venösen Zweig umfaßt. Bei der extrakorporalen Blutbehandlung besteht die Gefahr, dass infolge einer Leckage in den Blutkreislauf eindringende Luft zu einer lebensbedrohlichen Embolie des Patienten führt. Daher finden zur Überwachung des extrakorporalen Blutkreislaufs Luftdetektoren Verwendung, die im Allgemeinen im venösen Zweig des Blutkreislaufs angeordnet sind.

Es sind Dialyseverfahren bekannt, bei denen der Zugang zum Patienten mittels einer gemeinsamen Nadel, die sowohl mit dem arteriellen als auch venösen Zweig des Blutkreislaufs verbunden ist, oder mittels zweier Nadeln erfolgt, die jeweils mit dem arteriellen bzw. venösen Zweig des Blutkreislaufs verbunden sind.

Bei dem Einnadel-Dialyseverfahren kann für den Fall einer Leckage im Bereich der Nadel während der arteriellen Phase angesaugte Luft zum Teil im Bereich der Y-Verbindung zwischen dem arteriellen und venösen Zweig im Schlauchsystem verbleiben, wobei die Gefahr besteht, dass Teile dieser angesaugten Luft dem Patienten in der venösen Phase direkt zugeführt werden, ohne unmittelbar den Luftdetektor im venösen Zweig auszulöschen. Während nur einer Phase können beispielsweise 20 ml Luft angesaugt werden.

Bis die in der arteriellen Phase angesaugte Luft von dem Luftdetektor im venösen Zweig des Blutkreislaufs erkannt wird, können in Abhängigkeit von dem gewählten Schlagvolumen und der Größe des Dialysators viele Phasen vergehen, so dass es möglich ist, dass eine Fehlermeldung stark zeitverzögert ist.

Die bekannten Überwachungssysteme zur Detektion von Luft im extrakorporalen Blutkreislauf erfassen Änderungen der optischen, elektrischen oder akustischen Eigenschaften des im extrakorporalen Blutkreislauf strömenden Bluts. Beim Überschreiten bestimmter Grenzwerte wird Alarm ausgelöst und die weitere Blutzirkulation unterbrochen.

Neben den Überwachungssystemen zur Detektion von Luft sind Überwachungssysteme zur Detektion von Störungen des extrakorporalen Blutflusses, beispielsweise Stenosen bekannt.

Die DE 103 55 042 B3 beschreibt ein Verfahren zur Erkennung von Störungen des Blutflusses, bei dem der Phasenwinkel mindestens einer Oberschwingung eines sich im extrakorporalen Blutkreislaufs fortpflanzenden oszillierenden Drucksignals ermittelt wird, wobei Störungen des Blutflusses auf der Grundlage einer charakteristischen Veränderung des Phasenwinkels mindestens einer Oberschwingung des Drucksignals detektiert werden. Das bekannte Verfahren setzt voraus, dass die Blutpumpe ein oszillierendes Drucksignal im Blutkreislauf erzeugt.

Aus der US 2002/0174721 A1 ist ein Verfahren zur Erkennung von Stenosen in einem Schlauchleitungssystem während einer extrakorporalen Blutbehandlung bekannt, bei dem zum Erkennen einer Stenose das Frequenzspektrum eines oszillierenden Drucksignals analysiert wird, das auf den Betrieb der Blutpumpe zurückzuführen ist und sich im extrakorporalen Blutkreislauf fortpflanzt. Auf eine Stenose wird dann geschlossen, wenn sich die Dämpfung mindestens einer Oberschwingung des oszillierenden Drucksignals ändert.

Die DE 100 33 192 A1 beschreibt ein Verfahren zur Detektion arterieller Einlaufprobleme während einer extrakorporalen Blutbehandlung, bei der die Amplitude von periodischen Schwankungen des Drucks in der venösen Blutleitung gemessen und mit einem Grenzwert verglichen wird. Auf derartige Probleme wird bei Überschreiten des Grenzwertes geschlossen. Das bekannte Verfahren setzt voraus, dass die Blutpumpe im Blutkreislauf periodische Druckschwankungen erzeugt.

Der Erfindung liegt die Aufgabe zu Grunde, ein Verfahren anzugeben, mit dem sich mit hoher Sicherheit der Eintritt von Luft in ein Flüssigkeitssystem, insbesondere in den extrakorporalen Blutkreislauf einer Blutbehandlungsvorrichtung feststellen lässt. Eine weitere Aufgabe der Erfindung liegt darin, eine Vorrichtung bereitzustellen, die mit hoher Sicherheit die Erkennung eines Lufteintritts in ein Flüssigkeitssystem, insbesondere einen extrakorporalen Blutkreislauf erlaubt. Eine Aufgabe der Erfindung ist auch, eine extrakorporale Blutbehandlungsvorrichtung mit einer Vorrichtung zur Erkennung eines Lufteintritts in den extrakorporalen Blutkreislauf mit hoher Sicherheit zu schaffen.

Die Lösung dieser Aufgaben erfolgt erfindungsgemäß mit den in den Patentansprüchen 1, 8, 10, 16 und 18 angegebenen Merkmalen. Vorteilhafte Ausführungsformen der Erfindung sind Gegenstand der Unteransprüche.

Bei dem erfindungsgemäßen Verfahren und der erfindungsgemäßen Vorrichtung werden periodische Druckschwankungen im Flüssigkeitssystem gemessen und analysiert. Das erfindungsgemäße Verfahren bzw. die erfindungsgemäße Vorrichtung beruhen auf der spektralen Zerlegung des gemessenen periodischen Drucksignals in ein System von Funktionen, wobei die Koeffizienten der Funktionen überwacht werden. Auf den Eintritt von Luft in das Flüssigkeitssystem wird geschlossen, wenn mindestens eine der Koeffizienten der Funktionen vorgegebene Grenzwerte über- bzw. unterschreitet

Die vorgegebenen Grenzwerte definieren einen Grenzwertbereich mit einem oberen und einem unteren Grenzwert, wobei die Koeffizienten der Funktionen jeweils mit dem oberen und unteren Grenzwert verglichen werden.

In Versuchen hat sich gezeigt, dass in ein Flüssigkeitssystem eindringende Luft, beispielsweise die aufgrund einer Leckage in einen extrakorporalen Blutkreislauf einer Blutbehandlungsvorrichtung eindringende Luft, kleine Druckschwankungen verursacht, deren Amplitude aber zu gering ist, um die vorgegebenen Grenzwerte der in Blutbehandlungsvorrichtungen im Allgemeinen vorhandenen Drucküberwachungssysteme zu über- bzw. unterschreiten. Die spektrale Zerlegung des periodischen Drucksignals lässt jedoch derartige Druckschwankungen deutlich werden, so dass auch die Detektion von nur geringen Mengen an Luft mit hoher Sicherheit möglich ist, die in das Flüssigkeitssystem, insbesondere den extrakorporalen Blutkreislauf einer Blutbehandlungsvorrichtung, insbesondere Dialysevorrichtung, eindringen.

Bei einem Flüssigkeitssystem, das einen Zweig aufweist, in dem eine Pumpe zum Fördern der Flüssigkeit angeordnet ist, werden die periodischen Druckschwankungen in dem Zweig des Flüssigkeitssystems stromauf der Pumpe gemessen. Der Zweig des Flüssigkeitssystems, in dem die periodischen Druckschwankungen gemessen werden, kann eine die Flüssigkeit führende Leitung sein.

Grundsätzlich reicht es aus, wenn nur einer der Koeffizienten der Funktionen überwacht wird. Die Sicherheit der Detektion eines Lufteintritts kann aber dadurch erhöht werden, dass mehrere Koeffizienten der Funktionen überwacht werden. Auf einen Lufteintritt wird dann geschlossen, wenn sämtliche der mehreren Koeffizienten der Funktionen die vorgegebenen Grenzwerte über- bzw. unterschreiten.

Das erfindungsgemäße Verfahren bzw. die erfindungsgemäße Vorrichtung sieht vorzugsweise die Zerlegung des periodischen Drucksignals in ein System von orthogonalen, komplexen Funktionen bzw. komplex, konjugierten Funktionen vor, beispielsweise in ein System von Sinusfunktionen und Cosinusfunktionen.

Die Erkennung des Lufteintritts auf der Grundlage der spektralen Zerlegung des periodischen Drucksignals in ein System von Funktionen bietet insbesondere bei der Detektion eines Lufteintritts in den arteriellen Zweig des extrakorporalen Blutkreislaufs einer extrakorporalen Blutbehandlungsvorrichtung nach dem Einnadel-Dialyseverfahren Vorteile. Zur Erkennung eines Lufteintritts in den extrakorporalen Blutkreislaufs werden die Druckschwankungen vorzugsweise im arteriellen Zweig stromauf der im arteriellen Zweig des extrakorporalen Blutkreislaufs im Allgemeinen angeordneten Blutpumpe gemessen und analysiert. Bei dem Einnadel-Dialyseverfahren kann ein Lufteintritt in den extrakorporalen Blutkreislauf somit bereits stromauf der Blutpumpe erfolgen, so dass während des Bruchteils einer Phase die Förderung des Bluts gestoppt und ein akustischer und/oder optischer Alarm als Hinweis auf einen Lufteintritt ausgelöst werden kann. Auch beim Zweinadel-Dialyseverfahren kann ein Lufteintritt im arteriellen Zweig erkannt werden. Die Detektion eines Lufteintritts kann auch als Indikator für die Generation von Mikroblasen verwendet werden, die bei den bekannten

Blutbehandlungsvorrichtungen im Allgemeinen mit einem Luftdetektor im venösen Zweig des extrakorporalen Blutkreislaufs detektiert werden.

Im Folgenden wird ein Ausführungsbeispiel der Erfindung unter Bezugnahme auf die Zeichnungen näher erläutert.

Es zeigen:

Fig. 1 die wesentlichen Komponenten einer Blutbehandlungsvorrichtung zusammen mit einer Vorrichtung zur Erkennung des Eintritts von Luft in den extrakorporalen Blutkreislauf in stark vereinfachter schematischer Darstellung und

Fig. 2 die Intensität der Grundschwingung sowie der ersten und zweiten Oberschwingung des gemessenen periodischen Drucksignals als Funktion der Zeit.

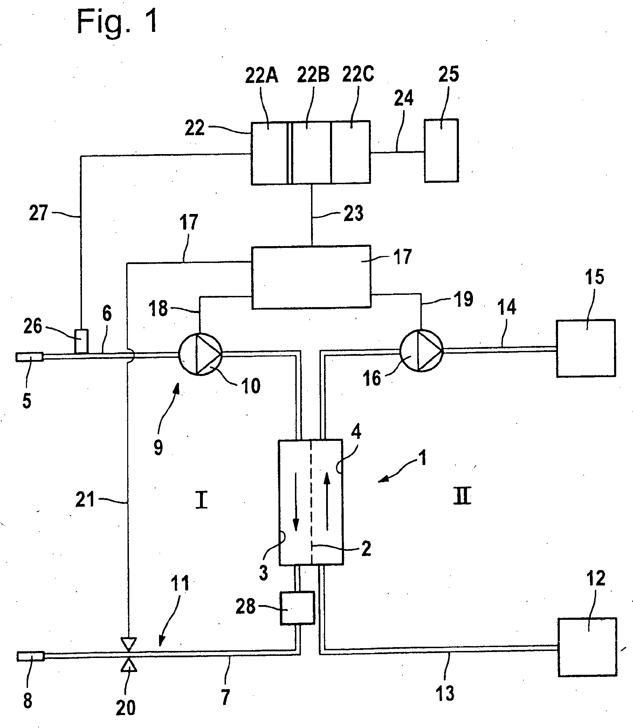

Figur 1 zeigt die wesentlichen Komponenten einer extrakorporalen Blutbehandlungsvorrichtung, im vorliegenden Ausführungsbeispiel einer Hämodialysevorrichtung, die über eine Vorrichtung zur Erkennung des Eintritts von Luft in den extrakorporalen Blutkreislauf, insbesondere den arteriellen Zweig des extrakorporalen Blutkreislaufs verfügt. Die Hämodialysevorrichtung weist einen Dialysator 1 auf, der durch eine semipermeable Membran 2 in eine Blutkammer 3 und eine Dialysierflüssigkeitskammer 4 unterteilt ist. An das Gefäßsystem des Patienten ist mittels einer arteriellen Punktionskanüle 5 eine arterielle Schlauchleitung 6 angeschlossen, die zu dem Einlass der Blutkammer 3 des Dialysators 1 führt. Von dem Auslass der Blutkammer 3 des Dialysators 1 geht eine venöse Schlauchleitung 7 ab, die mittels einer venösen Punktionskanüle 8 an das Gefäßsystem des Patienten angeschlossen ist. Im arteriellen Zweig 9 des extrakorporalen Blutkreislaufs I ist eine okkludierende Blutpumpe 10, insbesondere Rollenpumpe, angeordnet, während im venösen Zweig 11 des extrakorporalen Blutkreislaufs I ein Blasenfänger 28, beispielsweise eine Tropfkammer, angeordnet ist.

Bei der beschriebenen Dialysevorrichtung handelt es sich um eine Dialysevorrichtung für das Zweinadel-Dialyseverfahren. Eine Dialysevorrichtung für das Einnadel-Dialyseverfahren unterscheidet sich von der Zweinadel-Dialysevorrichtung im Wesentlichen nur dadurch, dass die arterielle und venöse Blutleitung mittels einer Y-Kopplung zusammengeführt sind, wobei das Blut dem Patienten über nur eine Nadel in aufeinanderfolgenden Phasen entnommen und zugeführt wird. Darüber hinaus kann die Einnadel-Dialysevorrichtung noch über eine Ausgleichskammer verfügen, die stromab der arteriellen Blutpumpe 10 angeordnet ist, sowie eine weitere Blutpumpe, die stromab der Ausgleichskammer im arteriellen Zweig 9 des extrakorporalen Blutkreislaufs 1 angeordnet ist.

Im Folgenden wird die Erfindung unter Bezugnahme auf die Zweinadel-Dialysevorrichtung beschrieben, wobei periodische Druckschwankungen stromauf der arteriellen Blutpumpe 10 gemessen und analysiert werden. Auch bei der Einnadel- Dialysevorrichtung werden die periodischen Druckschwankungen stromauf der arteriellen Blutpumpe 10 gemessen.

Der Dialysierflüssigkeitskreislauf II der Hämodialysevorrichtung umfaßt eine Dialysierflüssigkeitsquelle 12, an der eine Dialysierflüssigkeitszuführleitung 13 angeschlossen ist, die zu dem Einlass der Dialysierflüssigkeitskammer 4 des Dialysators 1 führt. Von dem Auslass der Dialysierflüssigkeitskammer 4 des Dialysators 1 geht eine Dialysierflüssigkeitsabführleitung 14 ab, die zu einem Auslass 15 führt. In die Dialysierflüssigkeitsabführleitung 14 ist eine Dialysierflüssigkeitspumpe 16 geschaltet.

Die Steuerung der Dialysevorrichtung übernimmt eine zentrale Steuereinheit 17, die über Steuerleitungen 18, 19 die Blut- und Dialysierflüssigkeitspumpe 10, 16 ansteuert. Stromab der Blutkammer 3 des Dialysators 1 befindet sich an der venösen Schlauchleitung 7 eine elektromagnetisch betätigbare Schlauchklemme 20, die über eine weitere Steuerleitung 21 von der zentralen Steuereinheit 17 geschlossen wird, wenn der Eintritt von Luft in den extrakorporalen Blutkreislauf erkannt wird. Darüber hinaus stoppt die Steuereinheit 17 noch die Blutpumpe 10. Zur Erkennung eines Lufteintritts weist die Dialysevorrichtung eine Vorrichtung 22 auf, die über eine Datenleitung 23 mit der zentralen Steuereinheit 17 kommuniziert. Die Vorrichtung 22 zur Erkennung eines Lufteintritts ist über eine weitere Datenleitung 24 mit einer Alarmeinheit 25 verbunden, die für den Fall eines Lufteintritts einen optischen und/oder akustischen Alarm gibt. Weiterhin steuert die Vorrichtung 22 bei einem Lufteintritt die zentrale Steuereinheit 17 an, die dann die venöse Schlauchklemme 20 schließt und die Blutpumpe 10 stoppt.

Nachfolgend wird das erfindungsgemäße Verfahren zur Erkennung eines Lufteintritts sowie der Aufbau und die Funktionsweise der Vorrichtung zur Erkennung eines Lufteintritts im Einzelnen beschrieben.

Die Vorrichtung 22 weist Mittel 22A zum Messen des Drucks im arteriellen Zweig 9 des extrakorporalen Blutkreislaufs I stromauf der arteriellen Blutpumpe 10 auf. Der Druck wird mittels eines in der arteriellen Schlauchleitung 6 stromauf der arteriellen Blutpumpe 10 angeordneten Drucksensors 26 gemessen, der über eine Datenleitung 27 mit der Vorrichtung 22 verbunden ist. Bei einem möglichen Lufteintritt aufgrund einer Leckage, beispielsweise infolge undichter Schlauchverbindungen, können Änderungen der durch die Blutpumpe 10 verursachten periodischen Druckschwankungen im arteriellen Zweig 9 des extrakorporalen Blutkreislaufs I stromauf der Blutpumpe 10 nachgewiesen werden.

Die Vorrichtung 22 verfügt über Mittel 22B, 22C zum Analysieren des gemessenen periodischen Drucksignals, um auf einen möglichen Lufteintritt zu schließen.

Die Mittel 22B, 22C zum Analysieren des periodischen Drucksignals umfassen Mittel 22B zum spektralen Zerlegen des Drucksignals in ein System von Funktionen. Die Mittel 22B zum Zerlegen des periodischen Drucksignals zerlegen das Drucksignal $P_{art}(t)$ in ein System von orthogonalen Funktionen, im vorliegenden Ausführungsbeispiel Sinusfunktionen und Cosinusfunktionen.

Grundsätzlich gilt für den Algorithmus der Luftüberwachung die Zerlegung des periodischen Drucksignals $P_{art}(t)$ in frequenzabhängige Koeffizienten $p(\omega)$.

Das Basissystem wird durch die orthogonalen, komplexen Funktionen $f(\omega,t)$ bzw. komplex konjugierten Funktionen $f'(\omega,t)$, aufgespannt.

$$[Gl.1] \qquad P_{art}(t) = \int f(\omega,t) \cdot p(\omega)d\omega$$

[G1.2] Beispiel für ein orthogonales Basissystem:

$$f(\omega,t) = e^{i\omega t} \, ; \int f(w,t) \cdot f^{\bullet}(\omega',t) = \delta(\omega',\omega)$$

Unter Berücksichtigung der Gln. 1 und 2 werden die Koeffizienten $p(\omega)$ berechnet nach:

$$\int f^{\bullet}(\omega,t) \cdot P_{art}(t)d\omega =$$

$$[\text{Gl.3}] \qquad \int f^{\bullet}(\omega,t) \cdot \int f(\omega',t) \cdot p(\omega')d\omega'd\omega =$$

$$p(\omega')\delta(\omega', \omega)=p(\omega)$$

Neben dem unter [G1.2] genannten Basissystem können auch andere Systeme verwendet werden.

Die frequenzabhängigen Koeffizienten $p(\omega)$ sind zeitlich stabil, sofern die Periodizität des arteriellen Drucksignals in Kongruenz zu der Frequenz $\omega$ des Algorithmus steht. Die Koeffizienten werden instabil, sobald Störungen hinzukommen, welche nicht phasenkorreliert zur Periodizität des arteriellen Drucksignals stehen. Diese Störungen können spontane Lufteintragungen in das arterielle Schlauchsystem stromaufwärts des arteriellen Druckaufnehmers sein.

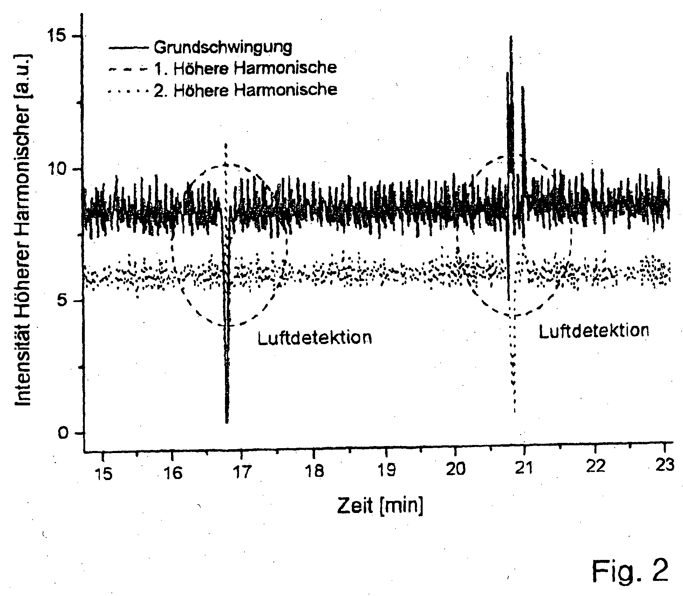

Figur 2 zeigt die Amplitude der Grundschwingung sowie der beiden ersten Oberschwingungen bei einer spontanen arterienseitigen Luftzufuhr von 1-2 ml bei einer in vitro Dialysebehandlung. Es sind die drei Koeffizienten $p(\omega1)$, $p(\omega2)$ und $p(\omega3)$ der spektralen Zerlegung des periodischen Drucksignals zu erkennen, d.h. die Grundschwingung und die erste und zweite höhere Harmonische. Die Koeffizienten sind zeitlich stabil, sofern keine Störung vorliegt. Störungen durch in das arterielle Schlauchsegment eingedrungene Luft verursachen eine Instabilität der Koeffizienten, wie aus Figur 2 ersichtlich ist. Das Verhalten der Instabilität hängt von der Größe und Wirkung der injizierten Luft ab. Hier wird die Intensität der Koeffizienten überwacht. Anstelle der Intensität der Koeffizienten kann aber auch deren Phase überwacht werden.

Es hat sich gezeigt, dass nach einer Änderung mindestens einer oder mehrerer der Koeffizienten infolge eines Lufteintritts, d.h. wenn der mindestens eine oder mehrere der Koeffizienten über einen vorgegebenen Grenzwert angestiegen oder unter einen vorgegebenen Grenzwert abgefallen sind, der mindestens eine oder mehrere der Koeffizienten wieder den ursprüngliche Wert annehmen oder um einen Wert abfallen bzw. ansteigen, dessen Betrag kleiner als der Betrag der Änderung ist. Die Tendenz, die ursprünglichen Werte wieder anzunehmen, kann in vorteilhafter Weise als zusätzliches Kriterium herangezogen werden, um den Eintritt von Luft zu erkennen, da sich die eindringende Luft nicht homogen im Flüssigkeitssystem verteilt. Es kommt vielmehr zu wechselnden größeren und kleineren Anteilen der Luft in der Flüssigkeit. Hierzu wird die Änderung der Messwerte überwacht und nach einem vorgegebenen Zeitraum nach Auftreten der Änderung überprüft, ob zumindest einer der Koeffizienten kleiner bzw. größer als ein vorgegebener Grenzwert ist, der um einen bestimmten Betrag kleiner bzw. größer als der vorgegebene obere bzw. untere Grenzwert für die Überwachung der Koeffizienten ist.

Die Mittel 22B, 22C zum Analysieren des periodischen Drucksignals umfassen weiterhin Mittel 22C zum Überwachen der Koeffizienten $p(\omega)$ der Sinus- und Cosinusfunktionen.

Die Detektion einer Instabilität infolge eines Lufteintritts ist möglich durch Festlegung eines Grenzwertbereichs $crit_i$ um den Wert des i-ten Koeffizienten $p(\omega_i)$. Wird dieser Grenzwert über- bzw. unterschritten, so ist die Instabilität erkannt, so dass auf einen Lufteintritt geschlossen wird.

$$[\text{Gl.4}] \qquad |p(\omega_i)| > crit_i \; ; i=1,2,3\ldots,N$$

Beispielsweise wird die Intensität der Grundschwingung sowie der ersten und zweiten Oberschwingung (i=1,2,3) überwacht. Hierzu wird der jeweilige Koeffizient $p(\omega_i)$ mit einem oberen und unteren Grenzwert verglichen, wobei bei Überschreiten des oberen Grenzwertes oder Unterschreiten des unteren Grenzwertes darauf geschlossen wird, dass ein Lufteintritt vorliegt. Für den Fall eines Lufteintritts wird die Blutpumpe 10 gestoppt, die Schlauchklemme 20 geschlossen und ein akustischer und/oder optischer Alarm gegeben. Zusätzlich kann überwacht werden, ob zumindest einer der Koeffizienten nach Ablauf einer vorgegebenen Zeitdauer wieder einen vorgegebenen Wert erreicht hat, wobei dann nur für diesen Fall Alarm gegeben wird, ansonsten aber nicht auf einen Lufteintritt geschlossen wird.

Fehlalarme werden vorzugsweise dadurch ausgeschlossen, dass auf einen Lufteintritt nur dann geschlossen wird, wenn sämtliche Koeffizienten außerhalb des definierten Grenzwertbereichs liegen, d.h. größer oder kleiner als der obere bzw. untere Grenzwert sind. Mit zunehmender Anzahl der Koeffizienten wird die Wahrscheinlichkeit von Fehlalarmen geringer.

Wie in Fig. 2 zu erkennen ist, verändern sich die Koeffizienten bzgl. eines Wertes $p_{ref}(\omega_i)$, der nicht Null sein muss. Die jeweiligen Werte für die einzelnen Komponenten können als Konstanten in den Mitteln (22C) zum Überwachen der Koeffizienten gespeichert sein. Zweckmäßigerweise wird man die Werte aber über ein Zeitfenster gemittelt während der Anwendung laufend ermitteln, so dass sie den momentanen Gegebenheiten angepasst sind.

Es ergibt sich damit folgendes Kriterium, bei dem auf einen Lufteintritt geschlossen wird:

$$[Gl.5] \qquad |p(\omega_i) - p_{ref}(\omega_i)| > crit_i \; ; \; i=1,2,3\ldots,N$$

Es ist auch möglich, anstelle von absoluten Schranken relative Grenzwerte festzulegen, wobei verschiedene Kriterien für den oberen und unteren Grenzwert gelten können. In diesem Fall wäre auch das Vorzeichen der zwischen den Betragszeichen der Gleichung angeführten Differenz bei der Analyse der Werte zu berücksichtigen.

**Patentansprüche**

1. Verfahren zur Erkennung des Eintritts von Luft in ein Flüssigkeitssystem, insbesondere in einen extrakorporalen Blutkreislauf einer Blutbehandlungsvorrichtung, wobei der Druck im Flüssigkeitssystem gemessen und das gemessene Drucksignal analysiert wird,
**dadurch gekennzeichnet,**
**dass** periodische Druckschwankungen im Flüssigkeitssystem gemessen und das gemessene periodische Drucksignal spektral in ein System von Funktionen zerlegt wird, und dass die Koeffizienten der Funktionen überwacht werden, wobei auf einen Lufteintritt in das Flüssigkeitssystem geschlossen wird, wenn mindestens einer der Koeffizienten der Funktionen vorgegebene Grenzwerte über- bzw. unterschreitet.

2. Verfahren nach Anspruch 1, **dadurch gekennzeichnet, dass** das periodische Drucksignal spektral in ein System von orthogonalen Funktionen zerlegt wird.

3. Verfahren nach Anspruch 2, **dadurch gekennzeichnet, dass** die orthogonalen Funktionen Sinusfunktionen und Kosinusfunktionen sind.

4. Verfahren nach einem der Ansprüche 1 bis 3, **dadurch gekennzeichnet, dass** die vorgegebenen Grenzen einen Grenzwertbereich mit einem oberen und unteren Grenzwert definieren, wobei auf einen Lufteintritt in das Flüssigkeitssystem geschlossen wird, wenn mindestens einer der Koeffizienten der Funktionen den oberen Grenzwert überschreitet oder den unteren Grenzwert unterschreitet

5. Verfahren nach Anspruch 4, **dadurch gekennzeichnet, dass** mehrere Koeffizienten der Funktionen jeweils mit einem oberen und einem unteren Grenzwert verglichen werden, wobei auf einen Lufteintritt in das Flüssigkeitssystem geschlossen wird, wenn sämtliche der mehreren Koeffizienten der Funktionen den oberen Grenzwert überschreiten oder den unteren Grenzwert unterschreiten.

6. Verfahren nach einem der Ansprüche 1 bis 5, **dadurch gekennzeichnet, dass** das Flüssigkeitssystem einen Zweig aufweist, in dem eine Pumpe zum Fördern der Flüssigkeit angeordnet ist, wobei periodische Druckschwankungen in dem Zweig des Flüssigkeitssystems stromauf der Pumpe gemessen werden.

7. Verfahren nach Anspruch 6, **dadurch gekennzeichnet, dass** der Zweig des Flüssigkeitssystems, in dem periodische Druckschwankungen gemessen werden, eine die Flüssigkeit führende Leitung ist,

8. Verfahren nach einem der Ansprüche 1 bis 7 zur Erkennung des Eintritts von Luft in einen extrakorporalen Blutkreislauf einer Blutbehandlungsvorrichtung, die einen zu einer Blutbehandlungseinheit führenden arteriellen Zweig und einen von der Blutbehandlungseinheit abgehenden venösen Zweig aufweist, wobei die periodischen Druckschwankungen im arteriellen oder venösen Zweig des extrakorporalen Blutkreislaufs gemessen und analysiert werden.

9. Verfahren nach Anspruch 8, **dadurch gekennzeichnet, dass** die periodischen Druckschwankungen im arteriellen Zweig stromauf einer im arteriellen Zweig angeordneten Blutpumpe gemessen werden.

10. Vorrichtung zur Erkennung des Eintritts von Luft in ein Flüssigkeitssystem, insbesondere in einen extrakorporalen Blutkreislauf einer Blutbehandlungsvorrichtung, mit Mitteln (22A) zum Messen des Drucks in dem Flüssigkeitssystem und Mitteln (22B, 22C) zum Analysieren des gemessenen Drucksignals, wobei die Mittel (22B, 22C) zum Analysieren des Drucks Mittel (22B) zum spektralen Zerlegen des gemessenen periodischen Drucksignals in ein System von Funktionen und Mittel zum Überwachen (22C) der Koeffizienten der Funktionen aufweist, wobei die Mittel zum Überwachen der Koeffizienten der Funktionen derart ausgebildet sind, dass auf einen Lufteintritt in das Flüssigkeitssystem geschlossen wird, wenn mindestens einer der Koeffizienten der Funktionen vorgegebene Grenzwerte über- bzw. unterschreitet,
**dadurch gekennzeichnet, dass** die Mittel (22B, 22C) zum Überwachen der Koeffizienten der Funktionen Mittel zum Vergleichen aufweisen, die derart ausgebildet sind, dass mindestens einer der Koeffizienten mit einem oberen und unteren Grenzwert verglichen wird, wobei auf einen Lufteintritt in das Flüssigkeitssystem geschlossen wird, wenn der mindestens eine Koeffizient der Funktionen den oberen Grenzwert überschreitet oder den unteren Grenzwert unterschreitet.

11. Vorrichtung nach Anspruch 10, **dadurch gekennzeichnet, dass** die Mittel (22B) zum spektralen Zerlegen des periodischen Drucksignals derart ausgebildet sind, dass das periodische Drucksignal spektral in ein System von orthogonalen Funktionen zerlegt wird.

12. Vorrichtung nach Anspruch 11, **dadurch gekennzeichnet, dass** die orthogonalen Funktionen Sinusfunktionen und Kosinusfunktionen sind.

13. Vorrichtung nach Anspruch 12, **dadurch gekennzeichnet, dass** die Mittel zum Vergleichen derart ausgebildet sind, dass mehrere Koeffizienten der Funktionen jeweils mit einem oberen und einem unteren Grenzwert verglichen werden, wobei auf einen Lufteintritt in das Flüssigkeitssystem geschlossen wird, wenn sämtliche der mehreren Koeffizienten der Funktionen den oberen Grenzwert überschreiten oder den unteren Grenzwert unterschreiten.

14. Vorrichtung nach einem der Ansprüche 10 bis 13, **dadurch gekennzeichnet, dass** das Flüssigkeitssystem einen Zweig (9) aufweist, in dem eine Pumpe (10) zum Fördern der Flüssigkeit angeordnet ist, wobei die Mittel zum Messen des Drucks (22A) Mittel zum Messen von periodische Druckschwankungen in dem Zweig des Flüssigkeitssystems stromauf der Pumpe sind.

15. Vorrichtung nach Anspruch 14, **dadurch gekennzeichnet, dass** der Zweig des Flüssigkeitssystems, in dem periodische Druckschwankungen gemessen werden, eine die Flüssigkeit führende Leitung (6) ist.

16. Vorrichtung nach einem der Ansprüche 10 bis 15 zur Erkennung des Eintritts von Luft in einen extrakorporalen Blutkreislauf (I) einer extrakorporalen Blutbehandlungsvorrichtung, der einen zu einer Blutbehandlungseinheit (1) führenden arteriellen Zweig (9) und einen von der Blutbehandlungseinheit abgehenden venösen Zweig (11) aufweist, wobei die Mittel (22A) zum Messen des Drucks Mittel zum Messen von periodischen Druckschwankungen im arteriellen oder venösen Zweig (9,11) des extrakorporalen Blutkreislauf (I) sind.

17. Vorrichtung nach Anspruch 16, **dadurch gekennzeichnet, dass** die Mittel (22A) zum Messen des Drucks Mittel zum Messen von periodischen Druckschwankungen im arteriellen Zweig (9, 11) des extrakorporalen Blutkreislaufs (I) stromauf einer im arteriellen Zweig (9) angeordneten Blutpumpe (10) sind.

18. Extrakorporale Blutbehandlungsvorrichtung, insbesondere Dialysevorrichtung, mit einem extrakorporalen Blutkreislauf (I), der einen zu einer Blutbehandlungseinheit (1) führenden arteriellen Zweig (9) und einen von der Blutbehandlungseinheit abgehenden venösen Zweig (11) aufweist, **dadurch gekennzeichnet, dass** die Blutbehandlungsvorrichtung eine Vorrichtung (22) zur Erkennung des Eintritts von Luft in den extrakorporalen Blutkreislauf (I) nach Anspruch 16 oder 17 aufweist.

**Claims**

1. A method for detecting the entry of air into a fluid system, in particular into an extracorporeal blood circuit of a blood treatment device, wherein the pressure in the fluid system is measured and the measured pressure signal is analyzed, **characterized in that**
periodic pressure fluctuations in the fluid system are measured and the measured periodic pressure signal is spectrally analyzed in a system of functions; the coefficients of the functions are monitored; an air entry into the fluid system is determined to exist if at least one of the coefficients of the functions is above or below predetermined limits.

2. A method according to claim 1, **characterized in that** the periodic pressure signal is spectrally analyzed in a system of orthogonal functions.

**3.** A method according to claim 2, **characterized in that** the orthogonal functions are sine functions and cosine functions.

**4.** A method according to one of claims 1 to 3, **characterized in that** the predetermined limits define a threshold range with an upper and lower threshold and an air entry into the fluid system is determined to exist if at least one of the coefficients of the functions is above the upper threshold or below the lower threshold.

**5.** A method according to claim 4, **characterized in that** a plurality of coefficients of the functions are each compared with an upper and a lower threshold and an air entry into the fluid system is determined to exist if all of the plurality of coefficients of the functions is above the upper threshold or below the lower threshold.

**6.** A method according to one of claims 1 to 5, **characterized in that** the fluid system has a branch containing a pump for supplying the fluid and periodic pressure fluctuations in the branch of the fluid system upstream of the pump are measured.

**7.** A method according to claim 6, **characterized in that** the branch of the fluid system in which periodic pressure fluctuations are measured is a line that conveys the fluid.

**8.** A method according to one of claims 1 through 7 for detecting the entry of air into an extracorporeal blood circuit of a blood treatment device, which has an arterial branch leading to a blood treatment unit and a venous branch leading away from the blood treatment unit and the periodic pressure fluctuations in the arterial or venous branch of the extracorporeal blood circuit are measured and analyzed.

**9.** A method according to claim 8, **characterized in that** the periodic pressure fluctuations in the arterial branch are measured upstream of a blood pump contained in the arterial branch.

**10.** A device for detecting the entry of air into a fluid system, in particular into an extracorporeal blood circuit of a blood treatment device, having means (22A) for measuring the pressure in the fluid system and means (22B, 22C) for analyzing the measured pressure signal;
the means (22B, 22C) for analyzing the pressure are equipped with means (22B) for spectral analysis of the measured periodic pressure signal in a system of functions and means (22C) for monitoring the coefficients of the functions; and the means for monitoring the coefficients of the functions are embodied so that an air entry into the liquid system is determined to exist if at least one of the coefficients of the functions exceeds or undershoots predetermined thresholds,
**characterized in that** the means (22B, 22C) for monitoring the coefficients of the functions have comparison means that are embodied such that at least one of the coefficients is compared to an upper and lower threshold and an air entry into the liquid system is determined to exist if at least one of the coefficients of the functions exceeds the upper threshold or undershoots the lower threshold.

**11.** The device according to claim 10, **characterized in that** the means (22B) for spectral analysis of the periodic pressure signal are embodied so that the periodic pressure signal is spectrally analyzed in a system of orthogonal functions.

**12.** The device according to claim 11, **characterized in that** the orthogonal functions are sine functions and cosine functions.

**13.** The device according to claim 12, **characterized in that** the comparison means are embodied so that a plurality of coefficients of the functions are each compared to an upper and lower threshold and an air entry into the liquid system is determined to exist if all of the plurality of coefficients of the functions exceed the upper threshold or undershoot the lower threshold.

**14.** The device according to one of claims 10 through 13, **characterized in that** the liquid system has a branch (9) containing a pump (10) for supplying the liquid and the means (22A) for measuring the pressure are means for measuring periodic pressure fluctuations in the branch of the liquid system upstream of the pump.

**15.** The device according to claim 14, **characterized in that** the branch of the liquid system in which periodic pressure fluctuations are measured is a line (6) that conveys the liquid.

**16.** The device according to one of claims 10 through 15 for detecting the entry of air into an extracorporeal blood circuit (I) of an extracorporeal blood treatment device, which has an arterial branch (9) leading to a blood treatment unit (1) and a venous branch (11) leading away from the blood treatment unit and the means (22A) for measuring the pressure are means for measuring periodic pressure fluctuations in the arterial or venous branch (9, 11) of the extracorporeal blood circuit (I).

17. The device according to claim 16, **characterized in that** the means (22A) for measuring the pressure are means for measuring periodic pressure fluctuations in the arterial branch (9, 11) of the extracorporeal blood circuit (I) upstream of a blood pump (10) situated in the arterial or venous branch (9).

18. An extracorporeal blood treatment device, in particular a dialysis device, having an extracorporeal blood circuit (I), which has an arterial branch (9) leading to a blood treatment unit (1) and a venous branch (11) leading away from the blood treatment unit, **characterized in that** the blood treatment device has a device (22) for detecting an entry of air into the extracorporeal blood circuit (I) according to claim 16 or 17.

**Revendications**

1. Procédé de détection de l'entrée d'air dans un système de liquide, plus particulièrement dans une circulation sanguine extracorporelle d'un dispositif de traitement du sang, la pression du système de liquide étant mesurée et le signal de pression mesuré étant analysé,
**caractérisé en ce que**
les variations de pression périodiques du système de liquide sont mesurées et le signal de pression périodique mesuré est décomposé de manière spectrale en un système de fonctions et **en ce que** les coefficients des fonctions sont surveillés, une entrée d'air dans le système de liquide étant détectée lorsqu'au moins un des coefficients des fonctions passe au dessus ou passe en dessous de valeurs limites prédéterminées.

2. Procédé selon la revendication 1, **caractérisé en ce que** le signal de pression périodique est décomposé de manière spectrale en un système de fonctions orthogonales.

3. Procédé selon la revendication 2, **caractérisé en ce que** les fonctions orthogonales sont des fonctions sinus ou des fonctions cosinus.

4. Procédé selon l'une des revendications 1 à 3, **caractérisé en ce que** les limites prédéterminées définissent une plage de valeurs limites avec une valeur limite supérieure et une valeur limite inférieure, une entrée d'air dans le système de liquide étant détectée lorsqu'au moins un des coefficients des fonctions dépasse la valeur limite supérieure ou passe en dessous de la valeur limite inférieure.

5. Procédé selon la revendication 4, **caractérisé en ce que** plusieurs coefficients des fonctions sont comparés chacun à une valeur limite supérieure et une valeur limite inférieure, une entrée d'air dans le système de liquide étant détectée lorsque tous ou une partie des plusieurs coefficients des fonctions dépassent la valeur limite supérieure ou passent en dessous de la valeur limite inférieure.

6. Procédé selon l'une des revendications 1 à 5, **caractérisé en ce que** le système de liquide présente un embranchement dans laquelle se trouve une pompe pour le transport du liquide, les variations périodiques de pression dans l'embranchement du système de liquide étant mesurées en amont de la pompe.

7. Procédé selon la revendication 6, **caractérisé en ce que** l'embranchement du système de liquide, dans lequel les variations périodiques de pression sont mesurées, est une conduite transportant le liquide.

8. Procédé selon l'une des revendications 1 à 7, pour la détection de l'entrée d'air dans une circulation sanguine extracorporelle d'un dispositif de traitement du sang, qui comprend un embranchement artériel conduisant vers une unité de traitement de sang et un embranchement veineux partant de l'unité de traitement de sang, les variations périodiques de pression dans l'embranchement artériel ou veineux de la circulation sanguine extracorporelle étant mesurées et analysées.

9. Procédé selon la revendication 8, **caractérisé en ce que** les variations périodiques de pression dans l'embranchement artériel sont mesurées en amont d'une pompe à sang disposée dans l'embranchement artériel.

**10.** Dispositif de détection de l'entrée d'air dans un système de liquide, plus particulièrement dans une circulation sanguine extracorporelle d'un dispositif de traitement de sang, avec des moyens (22A) pour la mesure de la pression dans le système de liquide et des moyens (22B, 22C) pour l'analyse du signal de pression mesuré,
les moyens (22B, 22C) pour l'analyse de la pression comprenant des moyens (22B) pour la décomposition spectrale du signal de pression périodique mesuré en un système de fonctions et des moyens (22C) pour la surveillance des coefficients des fonctions, les moyens de surveillance des coefficients des fonctions étant conçus de façon à ce qu'une entrée d'air dans le système de liquide soit détectée lorsqu'au moins un des coefficients des fonctions passe au dessus ou passe en dessous de valeurs limites prédéterminées,
**caractérisé en ce que** les moyens (22B, 22C) de surveillance des coefficients des fonctions comprennent des moyens de comparaison conçus de façon à ce qu'au moins un des coefficients soit comparé avec une valeur limite supérieure et une valeur limite inférieure, une entrée d'air dans le système de liquide étant détectée lorsqu'au moins un coefficient des fonctions dépasse la valeur limite supérieure ou passe en dessous de la valeur limite inférieure.

**11.** Dispositif selon la revendication 10, **caractérisé en ce que** les moyens (22B) pour la décomposition spectrale du signal de pression périodique sont conçus de façon à ce que le signal de pression périodique soit décomposé de manière spectrale en un système de fonctions orthogonales.

**12.** Dispositif selon la revendication 11, **caractérisé en ce que** les fonctions orthogonales sont des fonctions sinus ou des fonctions cosinus.

**13.** Dispositif selon la revendication 12, **caractérisé en ce que** les moyens de comparaison sont conçus de façon à ce que plusieurs coefficients des fonctions sont comparés chacun avec une valeur limite supérieure et une valeur limite inférieure, une entrée d'air dans le système de liquide étant détectée lorsque tous les coefficients des fonctions dépassent la valeur limite supérieure ou passent en dessous de la valeur limite inférieure.

**14.** Dispositif selon l'une des revendications 10 à 13, **caractérisé en ce que** le système de liquide comprend un embranchement (9) dans lequel se trouve une pompe (10) pour le transport du liquide, les moyens de mesure de la pression (22A) étant des moyens de mesure de variations périodiques de pression dans l'embranchement du système de liquide en amont de la pompe.

**15.** Dispositif selon la revendication 14, **caractérisé en ce que** l'embranchement du système de liquide, dans lequel les variations périodiques de la pression sont mesurées, est une ligne (6) conduisant un liquide.

**16.** Dispositif selon l'une des revendications 10 à 15, pour la détection de l'entrée d'air dans une circulation sanguine extracorporelle (I) d'un dispositif de traitement de sang extracorporel, qui comprend un embranchement artériel (9) conduisant vers une unité de traitement de sang (1) et un embranchement veineux (11) partant de l'unité de traitement de sang, les moyens (22A) pour la mesure de la pression étant des moyens de mesure des variations périodiques de pression dans l'embranchement artériel ou veineux (9, 11) de la circulation sanguine extracorporelle (I).

**17.** Dispositif selon la revendication 16, **caractérisé en ce que** les moyens (22A) pour la mesure de la pression sont des moyens de mesure des variations périodiques de pression dans l'embranchement artériel (9, 11) de la circulation sanguine extracorporelle (I) en amont d'une pompe à sang (10) disposée dans l'embranchement artériel (9).

**18.** Dispositif de traitement de sang extracorporel, plus particulièrement dispositif de dialyse, avec une circulation sanguine extracorporelle (I), qui comprend un embranchement artériel (9) conduisant vers une unité de traitement de sang (1) et un embranchement veineux (11) partant de l'unité de traitement de sang, **caractérisé en ce que** le dispositif de traitement de sang comprend un dispositif (22) pour la détection de l'entrée d'air dans la circulation sanguine extracorporelle (I) selon la revendication 16 ou 17.

# Fig. 1

Fig. 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10355042 B3
- US 20020174721 A1
- DE 10033192 A1